# EUROPEAN PATENT APPLICATION

(11) **EP 2 700 431 A1**
(43) Date of publication of application: **26.02.2014**
(21) Application number: 12190379.3
(22) Date of filing: 29.10.2012
(51) Int. Cl.: A61P 29/00, A61K 36/22, A61K 8/00, A61K 36/50, A61K 36/70

(54) **Plant extracts for modulating TRPV1 function**

(30) Priority: 24.08.2012 EP 12181645
(71) Applicant: AnalytiCon Discovery GmbH, 14473 Postdam (DE); Biotechnology Research And Information Network (B.R.A.I.N.) AG, 64673 Zwingenberg (DE)
(72) Inventor: Haustedt, Lars, 14471 Potsdam (DE); Siems, Karsten, 14552 Michendorf (DE); Kluge, Grit, 14959 Tribbin (DE); Krohn, Michael, 64653 Lorsch (DE); Kleber, Alice, 64625 Bensheim (DE)
(74) Representative: Fabry, Bernd

(57) **Abstract**

Surprisingly, it was found that the administration of plant extracts is efficacious in the blockade of TRPV1 channels. In particular, plant extracts of *Mangifera indica* and/or *Fumaria officinalis* and/or *Rumex japonicus* are efficacious as skin irritation-reducing agent and as an antagonistic agent for TRPV1.

## Description

### Field of invention

The present invention relates to a composition and methods for treating pain and other conditions related to TRPV1 and their use in various areas of application.

### Background of the invention

A variety of ion channel proteins exist to mediate ion flux across cellular membranes. The proper expression and function of ion channel proteins is essential for the maintenance of cell function, intracellular communication, and the like. Numerous diseases are the result of misregulation of membrane potential or aberrant calcium handling. Given the central importance of ion channels in modulating membrane potential and ion flux in cells, identification of agents that can promote or inhibit particular ion channels are of great interest as research tools and as possible therapeutic agents.

Transient receptor potential (TRP) channels are known to be at the forefront of mammals sensory systems, and have been found to be involved in the response to temperature, touch, pain, osmolarity, pheromones, taste and other stimuli. It is thought that the role of TRP channels is by far broader than simple sensory transduction as they are able to respond to many stimuli from both inside and outside of the cell.

Mammals are able to detect temperature with specialised neurons in their peripheral nervous system. These neurones are a subset of TRP channels: vanilloid-type channels (TRPV). Four different TRPV channels (TRPV1 to 4) have been identified which are implicated in heat sensing. These are temperature sensitive ion channels and are critical contributors to normal pain and temperature sensation. As such they are useful targets for the relief of pain.

Transient receptor potential vanilloid type 1 (TRPV1) receptor is a ligand-gated nonselective cation channel activated by heat (typically above 43 °C), low pH (less than 6)and endogenous lipid molecules such as anandamide, N-arachidonoyl-dopamine, N-acyl-dopamines and products of lipoxygenases (e.g., 12- and 15-(S)-HPETE(12- and 15-S-hydroperoxy-5Z, 8Z, 10E, 14Z-eicosatetraenoic acid)) termed endovanilloids. Apart from peripheral primary afferent neurons and dorsal root ganglia, TRPV1 receptor is expressed throughout the brain. Recent evidence shows that TRPV1 receptor stimulation by endocannabinoids or by capsaicin leads to analgesia and this effect is associated with glutamate increase and the activation of OFF cell population in the rostral ventromedial medulla (RVM).

TRPV1 is also expressed in many non-neuronal cells, among them keratinocytes, differentiated sebocytes, hair follicle cells, sweat gland ducts, the secretory portion of eccrine sweat glands, mast cells and 3T3-L1-preadipocyt.es and fibroblasts. TRPV1 activation in keratinocytes by capsaicin or heat causes the release of inflammatory mediators such as PGE2 (prostaglandin E2) and IL-8 (interleukin-8), and induction of matrix metalloproteinases such as MMP-1, respectively.

TRPV1 has also been found to be involved in the regulation of body temperature, anxiety and mediation of long term depression (LTD) in the hippocampus. TRPV1 channels are also located on sensory afferents which innervate the bladder. Inhibition of TRPV1 has been shown to ameliorate urinary incontinence symptoms.

TRPV1 modulators have been described in, for example, WO 2007/054480, which teaches the effect of 2-(benzimidazol-1-yl)-acetamide derivatives in the treatment of TRPV1 related diseases. WO 2008/079683 teaches compounds being a conjugated two ring system of cyclohexyl and phenyl for inhibiting TRPV1 receptor. EP01939173 teaches O-substituted-dibenzyl urea- or thiourea- derivatives as TRPV1 receptor antagonists. WO 2008/076752 teaches benzoimidazole compounds as potent TRPV1 modulators and EP01908753 teaches TRPVI modulators being heterocyclidene acetamide derivatives.

Therefore, the problem underlying the present invention has been to develop new modulators which exhibit the properties of blocking transient receptor potential vanilloid type 1 (TRPV1) receptor. It has therefore been the object of the present invention to develop new agents which act as TRPV1 antagonists and that reduce skin irritation, especially reduce or alleviate one or more human skin sensations like stinging, burning, tingling, tickling and skin tightness and further preventing or treating acute pain, chronic pain, touch sensitivity, itching sensitivity, inflammation, fever, hot flashes, rheumatoid arthritis.

This object is achieved by using plant extracts from *Mangifera indica and*/*or Fumaria officinalis and*/*or Rumex japonicus* as skin irritation-reducing agent. These plant extracts show an antagonistic effect against TRPV1, thus the extracts from *Mangifera indica and*/*or Fumaria officinalis and*/*or Rumex japonicus* can be formulated in cosmetic formulations and pharmaceutical preparations for the treatment or prevention of skin irritation.

### Description of the invention

Surprisingly, it was found that the administration of plant extracts is efficacious in the blockade of TRPV1 channels. In particular, plant extracts of *Mangifera indica* and/or *Fumaria officinalis* and/or *Rumex japonicus* are efficacious as skin irritation-reducing agent and as an antagonistic agent for TRPV1.

The present invention therefore refers to a composition obtainable by the method of extracting plant materials selected from the group consisting of *Mangifera indica* and/or *Fumaria officinalis* and/or *Rumex japonicus.*

The composition which is a plant extract of *Mangifera indica* and/or *Fumaria officinalis* and/or *Rumex japonicus* is obtainable, in a preferred embodiment, by the method comprising the steps of:
(a) aqueous and / or alcoholic extraction of plant materials selected from the group consisting of *Mangifera indica and*/*or Fumaria officinalis and*/*or Rumex japonicus;*
(b) sonicating the mixture thus obtained; and
(c) centrifugating the mixture thus obtained.

In this context "plant materials" refers to parts of the respective plant of *Mangifera indica and*/*or Fumaria officinalis and*/*or Rumex japonicus.* The plant materials may include parts of the whole plant selected from the group consisting of blossoms, fruits, buds, roots, seeds and/or leaves or the whole plant itself.

Surprisingly, it has been observed that using a composition which comprises the plant extracts according to the invention skin irritation, especially stinging, burning, tingling, tickling and skin tightness can be reduced. Another advantage of the present invention is that the composition of the present invention are useful for preventing or treating acute pain, chronic pain, touch sensitivity, itching sensitivity, inflammation, fever, hot flashes, rheumatoid arthritis. This allowing the use of the composition which comprises plant extracts of *Mangifera indica and*/*or Fumaria officinalis and*/*or Rumex japonicus* as an antagonistic agent for TRPV1.

The plant extracts of *Mangifera indica and*/*or Fumaria officinalis and*/*or Rumex japonicus* have skin soothing effects on sensitive skin. In the case of humans with delicate, sensitive or easily injured skin, a phenomenon called "stinging" sensations ("to sting" = becoming injured, burn, be painful) can be observed. Typical adverse phenomena associated with the terms "stinging" or "sensitive skin" are reddening of the skin, tingling, prickling, skin tightness and burning of the skin. They can be caused by stimulating environmental conditions, such as e.g. massage, action of surfactants, influence of weather, such as heat, cold, dryness and also damp heat, thermal radiation and UV radiation, e.g. from the sun, or psychological stress.

The composition obtainable by the method of extracting plant materials selected from the group consisting of *Mangifera indica* and/or *Fumaria officinalis* and/or *Rumex japonicus* therefore may be formulated into pharmaceutical preparations or cosmetic formulations. Thus, the invention also relates to a pharmaceutical preparation and a cosmetic formulation comprising extracts of plants selected from the group consisting of *Mangifera indica* and/or *Fumaria officinalis* and/or *Rumex japonicus* for preventing, treating or alleviating symptoms of disease, disorder or condition involving activation of TRPV1.

The pharmaceutical preparation of the invention can be used for prophylaxis (prevention) of skin irritations and/or for treatment of skin irritations for medical and/or other than medical purposes. The pharmaceutical preparation of the invention can be used for the treatment of pain conditions in the field of human medicine, such as, for example, urticaria, contact dermatitis, atopy and generally all inflammation processes, including tooth and gum inflammations, such as parodontosis.

### Plant extracts

The composition obtainable by the method of extracting plant materials selected from the group consisting of *Mangifera indica* and/or *Fumaria officinalis* and/or *Rumex japonicas* is a plant extract which is obtained from plant materials from *Mangifera indica* or *Fumaria officinalis* or *Rumex japonicus* and mixtures thereof. These plant extracts from *Mangifera indica* or *Fumaria officinalis* or *Rumex japonicus* and mixtures thereof act as an antagonistic agent for TRPV1 and thus inhibit or reduce skin irritation, especially stinging, burning, tingling, tickling and skin tightness. Thus, in a preferred embodiment the plant extracts are provided in an antagonistically effective amount to the skin.

In the context of this invention an "antagonistic agent for TRPV1" refers to a pharmaceutical and/or cosmetic active inhibition of the TRPV1 related bioactivity.

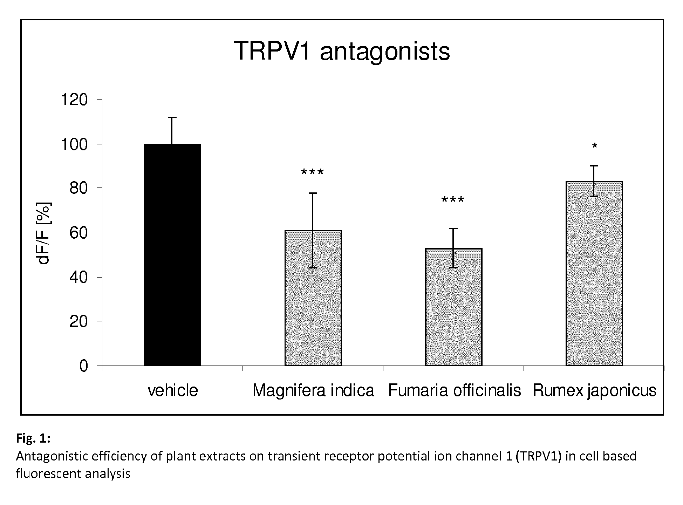

For the evaluation of skin irritation, a stimulation of skin irritation through a pure agonist, e.g. capsaicin is set as 100% (see Fig. 1, black column). In the context of this invention an "antagonistically effective amount" means an amount sufficient to modulate, and preferably reduce the TRPV1 receptor activity by at least about 30 percent, more preferably at least 50 or 75 percent, most preferably by at least 80 or 90 percent. Thus, the skin irritation itself is reduced to at least 10-20%, more preferably 35-50%, most preferably more than 70%.

The composition obtainable by the method of extracting plant materials of the present invention may comprise one or more active compounds which derived from plant materials of *Mangifera* or *Fumaria* or *Rumex, especially Mangifera indica* or *Fumaria officinalis* or *Rumex japonicus* or mixtures thereof. These active compounds are herewith classified in three different groups referring to as active agents group I, II and III.

Active agents group I is obtained by the method of extracting *Mangifera,* preferably *Mangifera indica* and comprises the compounds: pyridine-3-carbon acid, pantothenic acid, 4,5-bis(hydroxymethyl)- 2-methyl-3-pyridinole, 3-hydroxy-5-hydroxy- methyl-2-methyl-4-pyridine- carbaldehyde, 4-(aminomethyl)-5-hydroxy- 6-methyl-3-pyridine methanol, alpha-carotine, carotene, lycopine (5R)-5-[(1S)-1,2-dihydroxy-ethyl]-3,4-di-hydroxy-5-hydrofurane-2-one, α-tocopherole, flavone, flavonoids, anthraquinones, preferably emodin, chrysophanol, physicon; thiamine and a compound with the following structure (A):

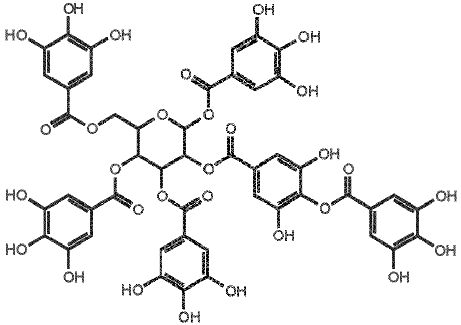

The active agents group I may also comprises as active compounds trace elements like potassium, calcium, copper, iron, manganese, magnesium and zinc.

Active agents group II is obtained by the method of extracting *Fumaria,* preferably *Fumaria officinalis* and comprises the compounds: alkaloides, preferably protoberberine, (-)-scoulerine; protopine; spirobenzyl-isochinoline, fumaricine, (+)-fumariline; indene-benzazepine, fumaritine, fumarofine; carboxy acids, preferably chlorogenic acid, fumaric acid, caffeic acid; flavonoid-glycosides, preferably quercetin-glycoside, ascorbic acid, flavanoids, tannin, and a compound with the following structure (B):

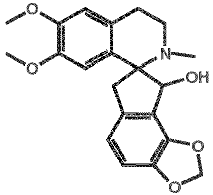

Active agents group III is obtained by the method of extracting *Rumex,* preferably *Rumex japonicus* and comprises the compounds: anthraquinones, preferably emodin, flavonoids, kaempferol-3-O-ß-d-glucoside, quercetin, quercitrin, isoquercitrin, catechin, flavones, preferably the ones listed in Table 1 below:

**Table 1: Active flavones from Rumex japonicus**

| **Species** | **Structure** | **R** | **R** | **R** | **R** | **R** |
|---|---|---|---|---|---|---|
| Apigenin | 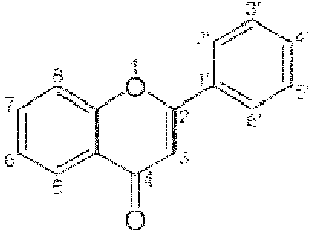 | OH | - | OH | - | OH |
| Acacetin | | OH | - | OH | - | OCH₃ |
| Luteolin | | OH | - | OH | OH | OH |
| Chrysin | | OH | - | OH | - | - |
| Chrysoeriol | | OH | - | OH | OCH₃ | OH |
| Diosmetin | | OH | - | OH | OH | OCH₃ |
| Tectochrysin | | OH | - | OCH₃ | | - |
| Scutellarein | | OH | OH | OH | - | OH |
| Eupatorin | | OH | OCH₃ | OCH₃ | OH | OCH₃ |
| Genkwanin | | OH | - | OCH₃ | OCH₃ - | OH |

In particular, pharmaceutical preparations or cosmetic formulations of the present invention comprise plant extracts selected from the group consisting of *Mangifera indica* or *Fumaria officinalis* or *Rumex japonicus* or mixtures thereof. These plant extracts preferably comprise one or more compounds of the above active agents group I or II or III which can act as an antagonistic agent for TRPV1, thus binding to TRPV1 and inhibit the activity of TRPV1. The compounds of the active agents group I or II or III used in pharmaceutical preparations or cosmetic formulations may be able to reduce or alleviate one or more human skin sensations from the group consisting of stinging, burning, tingling, tickling and skin tightness. In addition, the plant extracts comprising the active agents of group I or II or III prevent or treat diseases or conditions, especially chosen from the group of acute pain, chronic pain, touch sensitivity, itching sensitivity, inflammation, fever, hot flashes, and rheumatoid arthritis.

The extracts according to the present invention may be prepared by methods known per se, i.e. for example by aqueous, alcoholic or aqueous/alcoholic extraction of the plants or parts thereof. Suitable extraction processes are any conventional extraction processes, such as maceration, re-maceration, digestion, agitation maceration, vortex extraction, ultrasonic extraction, counter current extraction, percolation, re-percolation, evacolation (extraction under reduced pressure), diacolation and solid/liquid extraction under continuous reflux. Percolation is advantageous for industrial use. Any size reduction methods known to the expert, for example freeze grinding, may be used. Preferred solvents for the extraction process are organic solvents, water (preferably hot water with a temperature above 80 °C and more particularly above 95 °C or mixtures of organic solvents and water, more particularly low molecular weight alcohols with more or less high water contents. Extraction with methanol, ethanol and water-containing mixtures thereof is particularly preferred. The extraction process is generally carried out at about 20 to about 100 °C and preferably at about 50 to about 70 °C. In one preferred embodiment, the extraction process is carried out in an inert gas atmosphere to avoid oxidation of the ingredients of the extract. This is particularly important where extraction is carried out at temperatures above 40 °C. The extraction times are selected by the expert in dependence upon the starting material, the extraction process, the extraction temperature and the ratio of solvent to raw material, etc. After the extraction process, the crude extracts obtained may optionally be subjected to other typical steps, such as for example purification, concentration and/or decoloration. If desired, the extracts thus prepared may be subjected, for example, to the selective removal of individual unwanted ingredients. The extraction process may be carried out to any degree, but is usually continued to exhaustion. Typical yields (=extract dry matter, based on the quantity of raw material used) in the extraction of the starting materials are of the order of about 1 to about 20, %, preferably about 2 to about 15 and more preferably about 5 to about 10 % b.w. - calculated on the starting materials.

### Pharmaceutical preparations

A preferred embodiment is the use of a plant extract of *Mangifera indica* or *Fumaria officinalis* or *Rumex japonicus* or mixtures thereof, in the manufacture of a pharmaceutical preparation for preventing or treating diseases or conditions that are alleviated by blockade of one or more types of TRP channel, preferably TRPV1.

Therefore, a preferred embodiment of the present invention is the use of the plant extracts according to the present invention in pharmaceutical preparations. A pharmaceutical preparation of the invention comprises:
(i) the composition obtainable by the method of extracting plant materials selected from the group consisting of *Mangifera indica* and/or *Fumaria officinalis* and/or *Rumex japonicus.*
(ii) one or more pharmaceutically acceptable carriers and/or solvent, wherein the plant extract of step (i) is provided in a dosage form providing an amount effective to prevent, treat or alleviate symptoms of disease, disorder or conditions associated with TRPV1 activity.

In the context of this invention "an amount effective to prevent, treat or alleviate symptoms of disease, disorder or condition associated with TRPV1 activity" refers to a pharmaceutical and/or cosmetic active inhibition of the TRPV1 related bioactivity. This amount refers to an amount effective to reduce or alleviate skin irritation and/or to avoid skin irritation of human skin, in particular regarding skin sensations like stinging, burning, tingling and/or tightness. This amount is an amount sufficient to modulate, and preferably reduce by at least about 30 percent, more preferably at least 50 or 75 percent, most preferably by at least 80 or 90 percent, the TRPV1 receptor activity.

In a further preferred embodiment of the present invention the pharmaceutical preparation comprises further additives like: anti-inflammatory agents, anti-scarring agents, anti-psoriatic agents, anti-proliferative agents, anti-fungal agents, anti-viral agents or anti-septic agents or mixtures thereof.

Suitable pharmaceutically acceptable carriers must be acceptable in the sense of being compatible with the other ingredients of the formulation and being not deleterious to the recipient thereof. The pharmaceutical carrier employed shall be, preferably, a solid, a gel or a liquid. Preferred pharmaceutical solid carriers are lactose, terra alba, sucrose, talc, gelatin, agar, pectin, acacia, magnesium stearate, stearic acid and the like. Preferred liquid carriers are phosphate buffered saline solution, syrup, oil such as peanut oil and olive oil, water, emulsions, various types of wetting agents, sterile solutions and the like. Similarly, the carrier may include time delay material well known to the art, such as glyceryl mono- stearate or glyceryl di-stearate alone or with a wax. Further suitable carriers are well known in the art, see, e.g., Remington's Pharmaceutical Sciences, Mack Publishing Company, Easton, Pennsylvania.

Suitable solvents are, preferably, selected so as not to affect the biological activity of the combination. Preferred diluents are distilled water, physiological saline, Ringer's solutions, dextrose solution, and Hank's solution.

Suitable anti-inflammatory agents comprise one or more actives selected from the groups consisting of:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone; and/or
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone; and/or
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea.

The pharmaceutical preparation can include further components like pure substances, skin care agents and physiological cooling agents selected from the group consisting of:
(iv) pure substances, preferably alpha-bisabolol, apigenin, apigenin-7-glucoside, gingerols, shogaols, gingerdiols, dehydrogingerdiones, paradols, natural avenanthramides, non-natural avenanthramides, preferably dihydroavenanthramide D, boswellic acid, phytosterols, glycyrrhizin, glabridin and licochalcone A; and/or
(v) skin care agents, preferably skin moisture retention regulators or skin repair agents, preferably selected from the group consisting of sodium lactate, urea and derivatives, glycerol, propylene glycol, 1,2-pentanediol, 1,2-hexanediol and 1,2-octanediol, collagen, elastin or hyaluronic acid, diacyl adipates, petrolatum, urocanic acid, lecithin, allantoin, panthenol, phytantriol, lycopene, (pseudo-)ceramides, preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, cholesterol, phytosterols, chitosan, chondroitin sulfate, lanolin, lanolin esters, amino acids, vitamin E and derivatives (preferably tocopherol, tocopheryl acetate), alpha-hydroxy acids (preferably citric acid, lactic acid, malic acid) and derivatives thereof, mono-, di- and oligosaccharides, preferably glucose, galactose, fructose, mannose, laevulose and lactose, polysugars, such as beta-glucans, in particular 1,3-1,4- beta -glucan from oats, alpha-hydroxy-fatty acids, triterpenic acids, such as betulic acid or ursolic acid, and algae extracts; and/or
(vi) physiological cooling agents, preferably selected from the group consisting of menthone glycerol acetal (also known as Frescolat(R)MGA), menthyl lactate (also known as Frescolat(R)ML, menthyl lactate is preferably 1-menthyl lactate, in particular 1-menthyl I- lactate), substituted menthyl-3-carboxylic acid amides (e.g. menthyl-3-carboxylic acid N- ethylamide, also known as WS-3, Na-(L-menthanecarbonyl)glycine ethyl ester, also known as WS-5), 2-isopropyl-N-2,3-trimethylbutanamide (also known as WS-23), substituted cyclohexanecarboxylic acid amides, 3-menthoxypropane-1,2-diol, 2-hydroxyethyl menthyl carbonate, 2-hydroxypropyl menthyl carbonate, N-acetylglycine menthyl ester, isopulegol, menthyl hydroxycarboxylic acid esters (e.g. menthyl 3-hydroxybutyrate), monomenthyl succinate, monomenthyl glutarate, 2-mercaptocyclodecanone, menthyl 2- pyrrolidin-5-onecarboxylate, 2,3-dihydroxy-p-menthane, 3,3,5-trimethylcyclohexanone glycerol ketal, 3-menthyl 3,6-di- and - trioxaalkanoates, 3-menthyl methoxyacetate and icilin.

The pharmaceutical preparation of the present invention, preferably, comprises more than one of the aforementioned carriers or diluents as well as other components such as adjuvants or non-toxic, non-therapeutic, non-immunogenic stabilizers and the like.

The composition obtainable by the method of extracting plant materials selected from the group consisting of *Mangifera indica* and/or *Fumaria officinalis* and/or *Rumex japonicas* is a plant extract which may be formulated as pharmaceutically acceptable salt, e g. inorganic base. Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred. It should be recognized that the particular counter-ion forming a part of any salt of this invention is usually not of a critical nature, so long as the salt as a whole is pharmacologically acceptable and as long as the counter-ion does not contribute undesired qualities to the salt as a whole.

The pharmaceutical preparation of the present invention, preferably, comprises one or more compounds of the active agents group I, II and / or III. Preferably, the compounds are selected from: active agents group I: pyridine-3-carbon acid, pantothenic acid, 4,5-bis(hydroxymethyl)- 2-methyl-3-pyridinole, 3-hydroxy-5-hydroxy- methyl-2-methyl-4-pyridine- carbaldehyde, 4-(aminomethyl)-5-hydroxy- 6-methyl-3-pyridine methanol, alpha-carotine, carotene, lycopine (5R)-5-[(1S)-1,2-dihydroxy-ethyl]-3,4-di-hydroxy-5-hydrofurane-2-one, α-tocopherole, flavone, flavonoids, anthraquinones, emodin, chrysophanol, physicon; thiamine and / or a compound with the following structure (A): 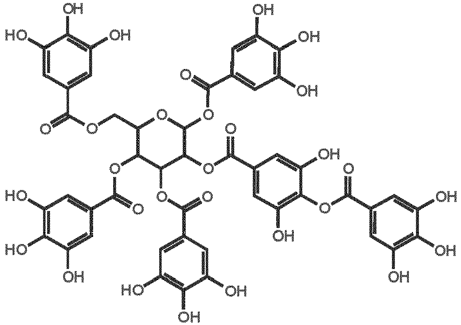 and / or active agents group II: alkaloides, preferably proto-berberine, (-) -scoulerine; protopine; spiro-benzyl-isochinoline, fumaricine, (+)-fumariline; indene-benzazepine, fumaritine, fumarofine; carboxy acids, preferably chlorogenic acid, fumaric acid, caffeic acid; flavonoid-glycosides, preferably quercetin-glycoside, ascorbic acid, flavanoids, tannin and / or a compound with the following structure (B): 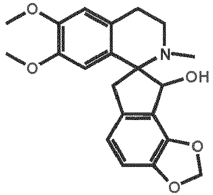 and / or active agents group III: anthraquinones, preferably emodin, flavonoids, kaempferol-3-O-ß-d-glucoside, quercetin, quercitrin, isoquercitrin, catechin and / or flavones. The pharmaceutical preparation comprises one or more compounds of the active groups I, II and / or III preferably reduces TRPV1 activity in skin, thus the compounds bind to TRPV1 and inhibit TRPV1 activity. A pharmaceutical preparation containing one or more compounds of active agents group I, II, and / or III, preferably, exhibits an antagonistic effect against TRPV1.

The pharmaceutical preparation of the present invention is, preferably, administered topically or orally. Suitable routes of administration conventionally used for drug administration are oral, intravenous, dermal or parenteral administration as well as inhalation. In some embodiments, it is advantageous to administer the preparation according to the present invention orally e.g. in the form of (compressed) tablets, dragees, comprimates, powders, capsules, juices, solutions and granules or in form of orally consumable products.

The pharmaceutical preparation of the present invention comprises the composition which is a plant extract of *Mangifera indica* and/or *Fumaria officinalis* and/or *Rumex japonicus* and that is obtainable by the above described extraction method, followed by a sonication and centrifugation step, and is preferably used for reducing TRPV1 activity in skin. Further, a pharmaceutical preparation of the present invention is preferably used for preventing, treating or alleviating symptoms of disease, disorder or condition involving activation of TRPV1.

The plant extracts of *Mangifera indica* and/or *Fumaria officinalis* and/or *Rumex japonicus* are preferably present in a pharmaceutical preparation from 0.1 to 10 % by weight, more preferably 0.1 to 5 % by weight and most preferred from 1 to 3% by weight of the pharmaceutical preparation.

### Cosmetic formulations

A preferred embodiment is the use of a plant extract, consisting of *Mangifera indica* or *Fumaria officinalis* or *Rumex japonicus* or mixtures thereof, in the manufacture of a cosmetic formulation for preventing or treating diseases or conditions that are alleviated by blockade of one or more types of TRP channel, preferably TRPV1.

Therefore, a preferred embodiment of the present invention is the use of the plant extracts according to the present invention in cosmetic formulations. The cosmetic formulation preferably comprises:
(i) the composition obtainable by the method of extracting plant materials selected from the group consisting of *Mangifera indica* and/or *Fumaria officinalis* and/or *Rumex japonicus*
(ii) one or more cosmetic acceptable carriers and/or solvent, wherein the plant extract of step (i) is provided in a dosage form providing an amount effective to prevent, treat or alleviate symptoms of disease, disorder or condition involving activation of TRPV1.

These plant extracts are, preferably obtained according to the above described extraction method, followed by a sonication and centrifugation step, wherein the extracts are obtained from plant materials selected form the group consisting of *Mangifera* or *Fumaria* or *Rumex* or mixtures thereof, preferably from plant materials of *Mangifera indica* or *Fumaria officinalis* or *Rumex japonicus* or mixtures thereof.

The cosmetical formulations according to the invention may further contain surfactants, oil bodies, solvents, carriers, emulsifiers, superfatting agents, pearlising waxes, consistency factors, polymers, silicone compounds, waxes, thickening agents, antiperspirant active ingredients, stabilizers, primary and secondary sun protection agents, antidandruff agents, biogenic agents, film formers, swelling agents, hydrotropes, enzyme inhibitors, anti-microbial agents, odour aborbers, preservatives, solubilizers, complexing agents, reducing agents, alkalising agents, perfume oils, fragrances, dyes and the like as additional auxiliaries and additives.

### Surfactants

Preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineraloladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Carriers, solvents and oil bodies

Particular preferred cosmetic formulations comprise one or more cosmetic acceptable carriers, solvents or oil bodies. These preparations are easy to handle and are stable over a long period of time, typically more than 3 months, preferably more than 6 months, without decomposition or crystallization of the compounds.

Suitable cosmetic acceptable **carriers and/or solvents** are selected from the group comprising of (i) (alkane) diols having 3 to 10 carbon atoms, preferably selected from the group consisting of 1,2-propylene glycol, 2-methylpropane-1,3-diol, 1,2-butylene glycol, 1,3- butanediol, 1,2-pentanediol, 1,3-pentanediol, 1,5-pentanediol, 2,4-pentanediol, 2-methyl- pentane-2,4-diol, 1,2-hexanediol, 1,6-hexanediol, 1,2-octanediol, dipropylene glycol, preferably 1,2-butylene glycol, 1,2-pentanediol and/or dipropylene glycol.

Suitable cosmetic acceptable ***oil bodies*** are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

Cosmetic formulations and compositions according to the present invention advantageously comprise a total amount of about 5 to about 70 % b.w., preferably about 7.5 to about 60 % b.w., more preferably about 10 to about 50 % b.w., even more preferably about 10 to about 40 % b.w. of the one or more (preferred) cosmetic acceptable carriers, solvents or oil bodies, in each case based on the total weight of the formulation or preparation.

### Emulsifiers

Other surfactants may also be added to the preparations as emulsifiers, including for example:
● products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
● C_{12/18} fatty acid monoesters and diesters of addition products of 1 to 30 mol ethylene oxide onto glycerol;
● glycerol mono- and diesters and sorbitan mono- and diesters of saturated and unsaturated fatty acids containing 6 to 22 carbon atoms and ethylene oxide addition products thereof;
● addition products of 15 to 60 mol ethylene oxide onto castor oil and/or hydrogenated castor oil;
● polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
● addition products of 2 to 15 mol ethylene oxide onto castor oil and/or hydrogenated castor oil;
● partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
● mono-, di and trialkyl phosphates and mono-, di- and/or tri-PEG-alkyl phosphates and salts thereof;
● wool wax alcohols;
● polysiloxane/polyalkyl polyether copolymers and corresponding derivatives;
● mixed esters of pentaerythritol, fatty acids, citric acid and fatty alcohol and/or mixed esters of C₆₋₂₂ fatty acids, methyl glucose and polyols, preferably glycerol or polyglycerol,
● polyalkylene glycols and
● glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
(i) Typical examples of suitable ***partial glycerides*** are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.
(ii) Suitable ***sorbitan esters*** are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.
(iii) Typical examples of suitable ***polyglycerol esters*** are Polyglyceryl-2 Dipolyhydroxy-stearate (Dehymuls^{®} PGPH), Polyglycerin-3-Diisostearate (Lameform TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.
(iv) Typical ***anionic emulsifiers*** are aliphatic C₁₂₋₂₂ fatty acids, such as palmitic acid, stearic acid or behenic acid for example, and C₁₂₋₂₂ dicarboxylic acids, such as azelaic acid or sebacic acid for example.
(v) Other suitable emulsifiers are ***zwitterionic surfactants**.* Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine , Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes and stabilizers

Besides natural oils used, ***waxes*** may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as ***stabilizers.***

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
● 3-benzylidene camphor or 3-benzylidene norcamphor and derivatives thereof, for example 3-(4-methylbenzylidene)-camphor;
● 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
● esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
● esters of salicylic acid, preferably salicylic acid-2-ethylhexyl ester, salicylic acid-4-isopropylbenzyl ester, salicylic acid homomenthyl ester;
● derivatives of benzophenone, preferably 2-hydroxy-4-methoxybenzophenone, 2-hydroxy-4-methoxy-4'-methylbenzophenone, 2,2'-dihydroxy-4-methoxybenzophenone;
● esters of benzalmalonic acid, preferably 4-methoxybenzalmalonic acid di-2-ethylhexyl ester;
● triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb^{®} HEB);
● propane-1,3-diones such as, for example, 1-(4-tert.butylphenyl)-3-(4'-methoxyphenyl)-propane-1,3-dione;
● ketotricyclo(5.2.1.0)decane derivatives.
Suitable water-soluble substances are
● 2-phenylbenzimidazole-5-sulfonic acid and alkali metal, alkaline earth metal, ammonium, alkylammonium, alkanolammonium and glucammonium salts thereof;
● sulfonic acid derivatives of benzophenones, preferably 2-hydroxy-4-methoxybenzophenone-5-sulfonic acid and salts thereof;
● sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, polyphenols, flavonoids, AHA acids, amino acids, ceramides, pseudoceramides, gingerols, shogaols, gingerdiols, dehydrogingerdiones, paradols, natural avenanthramides, non-natural avenanthramides, preferably dihydroavenanthramide D, boswellic acid, phytosterols, glycyrrhizin, and licochalcone A, urea, hyaluronic acid, allantoin, panthenol, lanolin, alpha-hydroxy acids (preferably citric acid, lactic acid), vitamin E and derivatives (preferably tocopherol, tocopheryl acetate), extracts or fractions from camomile, *Aloe vera,* oats, calendula, arnica, honeysuckle, rosemary, witch hazel, ginger or *Echinacea, Ginkgo biloba, Oleacea europensis, Glyzyrrhiza glabra, Vaccinium myrtillus, Trifolium pratense, Litchi sinensis, Vitis, vinifera, Brassica oleracea, Punica granatum, Petroselinium crispum, Centella asiatica, Passiflora incarnata, Medicago sativa, Melissa officinalis, Valeriana officinalis, Castanea sativa, Salix alba* and *Hapagophytum procumbens* extract, and vitamin complexes.

The composition may also comprise one or more of actives providing a benefit for the skin, in particular other skin irritation-reducing or skin-soothing agents, preferably selected from the group consisting of anti-inflammatory agents, physiological cooling agents, compounds that alleviate itching and/or compounds that alleviate reddening which are suitable for cosmetic and/or dermatological applications, wherein the one or more actives are preferably selected from the groups consisting of:
(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone; and/or
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone; and/or
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea,and/or
(iv) pure substances, preferably alpha-bisabolol, apigenin, apigenin-7-glucoside, gingerols, shogaols, gingerdiols, dehydrogingerdiones, paradols, natural avenanthramides, non-natural avenanthramides, preferably dihydroavenanthramide D, boswellic acid, phytosterols, glycyrrhizin, glabridin and licochalcone A; and/or
(v) skin care agents, preferably skin moisture retention regulators or skin repair agents, preferably selected from the group consisting of sodium lactate, urea and derivatives, glycerol, propylene glycol, 1,2-pentanediol, 1,2-hexanediol and 1,2-octanediol, collagen, elastin or hyaluronic acid, diacyl adipates, petrolatum, urocanic acid, lecithin, allantoin, panthenol, phytantriol, lycopene, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, cholesterol, phytosterols, chitosan, chondroitin sulfate, lanolin, lanolin esters, amino acids, vitamin E and derivatives (preferably tocopherol, tocopheryl acetate), alpha-hydroxy acids (preferably citric acid, lactic acid, malic acid) and derivatives thereof, mono-, di- and oligosaccharides, preferably glucose, galactose, fructose, mannose, laevulose and lactose, polysugars, such as beta - glucans, in particular 1,3-1,4- beta -glucan from oats, alpha-hydroxy-fatty acids, triterpenic acids, such as betulic acid or ursolic acid, and algae extracts; and/or
(vi) physiological cooling agents, preferably selected from the group consisting of menthone glycerol acetal, menthyl lactate preferably I-menthyl lactate, in particular I- menthyl I-lactate), substituted menthyl-3-carboxylic acid amides (e.g. menthyl-3- carboxylic acid N-ethylamide, Na-(L-menthanecarbonyl)glycine ethyl ester, 2-isopropyl-N- 2,3-trimethylbutanamide, substituted cyclohexanecarboxylic acid amides, 3- menthoxypropane-1,2-diol, 2-hydroxyethyl menthyl carbonate, 2-hydroxypropyl menthyl carbonate, N-acetylglycine menthyl ester, isopulegol, menthyl hydroxycarboxylic acid esters (e.g. menthyl 3-hydroxybutyrate), monomenthyl succinate, monomenthyl glutarate, 2-mercaptocyclodecanone, menthyl 2-pyrrolidin-5-onecarboxylate, 2,3-dihydroxy-p- menthane, 3,3,5-trimethylcyclohexanone glycerol ketal, 3-menthyl 3,6-di- and - trioxaalkanoates, 3-menthyl methoxyacetate and icilin.

### Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, perillic acid, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen^{®} CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

### Antiperspirant active ingredients

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulfate, sodium thiosulfate and the like may be present. Suitable alkalizing agents are ammonia, monoethanolamines, (L) arginine, AMP, etc.

### Fragrances and perfume oils

The compositions may also contain one or more of a ***fragrance,*** preferably having a Clog P value of at least 3, preferably of at least 4, more preferably of at least 5, and preferably selected from the group consisting of: alpha-amyl cinnamic aldehyde, alpha-hexyl cinnamic aldehyde, 2- phenoxyethylisobutyrate, methyl dihydrojasmonate, 4,6,6,7,8,8-hexamethyl-1,3,4,6,7,8- hexahydrocyclopenta[g]benzopyran, benzylsalicylate, 2-methyl-3-(4-tert-butylphenyl)propanal, 4,7-methano-3a,4,5,6,7,7a-hexahydro-5-indenyl acetate and/or 4,7-methano-3a,4,5,6,7,7a-hexahydro-6-indenyl acetate, styrallyl acetate, octahydro-2, 3,8,8-tetramethyl-2-acetonaphthone and/or 2-acetyl-1,2,3,4,6,7,8-octahydro-2,3,8,8- tetrame-thylnaphthaline, hexylsalicylate, 4-tert.-butylcyclohexyl acetate, 2-tert.- butylcyclohexyl acetate, alpha-ionone, 4-(4-hydroxy-4-methylpentyl)-3-cyclohexene carboxaldehyde, (E)-and/or (ZS-methylcyclopentadec-d-enone, 15-pentadec-11-enolide and/or 15-pentadec-12-enolide, Id-cyclopentadecanolide, 1 -(5,6,7, 8-tetrahydro- 3,5,5,6,8,8-hexamethyl-2-naphthalenyl)ethanone, ethylene brassylate, 2-ethyl-4-(2,2,3- trimethyl-3-cyclopenten-1-yl)-2-buten-l-ol, alpha-Santalol, 2,2-dimethyl-3-(3- methylphenyl)-propanol (Majantol), allyl heptanoate, 4-methylacetophenone, (4aR,5R,7aS,9R)-octahydro-2,2,5,8,8,9a-hexamethyl-4H-4a,9-methanoazuleno(5,6-d)- 1,3-dioxol), 1-(2,2,6-trimethylcyclohexyl)hexan-3-ol, benzylacetone, methyl cinnamate, 3a,6,6,9a-tetramethyldodecahydronaphtho[2,1-b]furan. The Clog P value (also known as log Pow) is the decimal logarithm of the distribution coefficient of a substance or material between 1-octanol to water. Clog P values are well known in the chemical arts as a calculated value that represents the relative affinity that a substance or material has for partitioning between octanol and water.

Suitable ***perfume oils*** are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, ·-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1% by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

The cosmetic formulation of the present invention may comprise one or more compounds of the active agents group I, II and / or III. Preferably, the compounds are selected from: active agents group I: pyridine-3-carbon acid, pantothenic acid, 4,5-bis(hydroxymethyl)- 2-methyl-3-pyridinole, 3-hydroxy-5-hydroxy- methyl-2-methyl-4-pyridine- carbaldehyde, 4-(aminomethyl)-5-hydroxy- 6-methyl-3-pyridine methanol, alpha-carotine, carotene, lycopine (5R)-5-[(1S)-1,2-dihydroxy-ethyl]-3,4-di-hydroxy-5-hydrofurane-2-one, α-tocopherole, flavone, flavonoids, anthraquinones, emodin, chrysophanol, physicon; thiamine and / or a compound with the following structure (A): 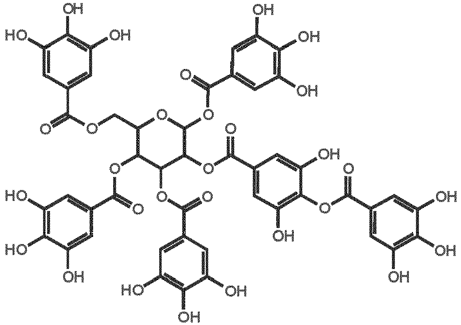 and / or active agents group II: alkaloides, preferably proto-berberine, (-) -scoulerine; protopine; spiro-benzyl-isochinoline, fumaricine, (+)-fumariline; indene-benzazepine, fumaritine, fumarofine; carboxy acids, preferably chlorogenic acid, fumaric acid, caffeic acid; flavonoid-glycosides, preferably quercetin-glycoside, ascorbic acid, flavanoids, tannin and / or a compound with the following structure (B): 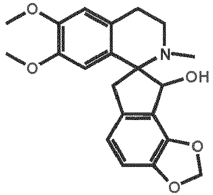 and / or active agents of group III: anthraquinones, preferably emodin, flavonoids, kaempferol-3-O-*ß*-d-glucoside, quercetin, quercitrin, isoquercitrin, catechin and / or flavones.

The cosmetic formulation of the present invention is preferably used for reducing TRPV1 activity in skin, thus inhibiting the activity of TRPV1 and acting as an antagonistic agent for TRPV1. The cosmetic formulation of the present invention comprises the composition which is a plant extract of *Mangifera indica* and/or *Fumaria officinalis* and/or *Rumex japonicus* and that is obtainable by the above described extraction method, followed by a sonication and centrifugation step, and is preferably used for reducing TRPV1 activity in skin. Further, a cosmetic formulation of the present invention is preferably used for preventing, treating or alleviating symptoms of disease, disorder or condition involving activation of TRPV1.

Preferably, the cosmetic formulation comprises one or more compounds of the active agents group I, II and / or III, wherein the compounds bind to TRPV1 and inhibit TRPV1 activity.

The cosmetic formulation can be a formulation for the region of the head, cleansing or care compositions for the face, face creams or lotions or ointments, sunscreen compositions, lipsticks or other lip cosmetics or lip care compositions.

The plant extracts of *Mangifera indica* and/or *Fumaria officinalis* and/or *Rumex japonicus* are preferably present in a cosmetic formulation from 0.1 to 10 % by weight, more preferably 0.1 to 5 % by weight and most preferred from 1 to 3% by weight of the cosmetic formulation.

In certain preferred embodiments, the pharmaceutical preparation and cosmetic formulation are administered by topical, transdermal or oral application. Preferably, the cosmetic formulation is administered topically or transdermally, most preferred topically.

Preferred pharmaceutical preparation and cosmetic formulation of to the invention comprising (preferred) cosmetically acceptable carriers and fragrance materials mentioned (in particular those with higher Clog P values). In particular in (topical) cosmetic composition and products, in particular those comprising water (preferably in an amount of 10 to 95 weight percent, more preferably 25 to 90 weight percent, even more preferably 40 to 90 weight percent, in each case based on the total weight of the composition or product), and in (topical) cosmetic composition and products comprising water and an oil phase (e.g. O/W or W/O-emulsions).

In a preferred embodiment the pharmaceutical preparation and cosmetic formulation is a topical O/W or W/O-emulsion formulation, which may be a micro-emulsion. The preparation and formulation comprises plant extracts of *Mangifera indica* and/or *Fumaria officinalis* and/or *Rumex japonicus* preferably in amount from 0.1 to 10 % by weight, more preferably 0.1 to 5 % by weight and most preferred from 1 to 3% by weight of the topic formulation.

Furthermore, the present invention relates to a method for treating or preventing a condition involving activation of TRPV1 or for which reduce TRPV1 activity can reduce severity of symptoms, comprising administering an effective amount of plant extract consisting of *Mangifera indica and*/*or Fumaria officinalis and*/*or Rumex japonicus.* In particular, the method to use to prevent, treat or alleviate symptoms of a disorder or condition is selected from the group consisting of acute pain, chronic pain, touch sensitivity, itching sensitivity, inflammation, fever, hot flashes, rheumatoid arthritis and stinging, burning, tingling, tickling and skin tightness of human skin. Furthermore, the prevention, treatment or alleviation of symptoms involving activation of TRPV1 is selected from the group consisting of acute pain, chronic pain, touch sensitivity, itching sensitivity, inflammation, fever, hot flashes, rheumatoid arthritis.

Preferably, the composition which is obtainable by the method of extracting plant materials selected from the group consisting of *Mangifera indica and*/*or Fumaria officinalis and*/*or Rumex japonicus* is for reducing TRPV1 activity in skin. Preferably, such a plant extract of the present invention is used as an antagonistic agent for TRPV1 and is used in an antagonistic effective amount for this purpose, which is usually an amount that is sufficient to modulate, and preferably reduce by at least about 30 percent, more preferably at least 50 or 75 percent, most preferably by at least 80 or 90 percent, the TRPV1 receptor activity.

The composition which is a plant extract of the plant materials selected from the group consisting of *Mangifera indica and*/*or Fumaria officinalis and*/*or Rumex japonicus* can act as an antagonistic agent for TRPV1 and therefore can be used as part of a prophylaxis or treatment for a variety of disorders and conditions, including, but not limited to, acute and/or chronic pain, touch sensitivity, burns, inflammation, diabetic neuropathy, psoriasis, eczema, dermatitis, post-herpetic neuralgia (shingles), migraine, incontinence, fever, hot flashes, osteoarthritis, oral mucositis, cancer pain, bladder cystitis, pain associated with Crohn's disease and Irritable Bowel Syndrome (IBS), rheumatoid arthritis, Grierson-Gopalan syndrome (better known as burning feet syndrome), burning mouth syndrome (BMS) and cough, or is used as a depilatory to promote loss of or inhibit the growth of hair on a patient.

The plant extracts of the present invention as an antagonistic agent for TRPV1 can be administered alone or in combination with other therapeutic agents, e.g., conjointly with one more of an anti-inflammatory agent, anti-acne agent, anti-wrinkle agent, anti-scarring agent, anti-psoriatic agent, anti-proliferative agent, anti-fungal agent, anti-viral agent, anti-septic agent, anti-migraine agent, keratolytic agent, or a hair growth inhibitor.

### Examples

The examples which follow are intended to illustrate the present invention without limiting the invention. Unless indicated otherwise all amounts, parts and percentages are based on the weight and the total amount or on the total weight and the total amount of the preparations.

### Example 1

### Extraction process

The dried and finely grounded plant material (*Magnifera indica, Fumaria officinalis, Rumex japonicas*) were extracted twice with fivefold ethanol in total (sonicated for about 15 minutes, shaken for about 30 minutes and afterwards centrifugated), yielding 15 to 5% extract regarding the quantity of plant material.

### Example 2

### Profiling of the plant extract by the following bioassay

The dried and finely grounded plant materials were extracted with MTB (methyl-tert-butylether) and methanol (ratio 1:1) and two times with methanol. The yielded extracts (of *Magnifera indica, Fumaria officinalis, Rumex japonicus*) was fractionated by RP (reversed-phase) HPLC.

Sample preparation: 1g extract were dissolved in 3 ml DMSO, 1 ml water were added following by filtration, afterwards liquid injection of the filtrate.

Method description:
Column: SelectB 12 µm 50x25 mm with pre-column
Detection: HPLC-ELSD-UV (254 nm)
Mobile Phase: A:H2O; B: ACN:MeOH (1:1)
Gradient: 10%B 0.5 min; from 10% B to 100% B in 0.8 min, 6.3 min 100% B
Flow: 28 ml/min

### Example 3

### Analytic LC/MS-Method to identify active agents in extracts

HPLC System: PE series 200
MS System: Applied Biosystems API 150, 165 or 365
Data System: Analyst 1.3 or Masschrom 1.2.1
Column: LiChrospher 60 RP select B 5 µm, 250x4mm, pre column 4x4mm
Flow Rate: 0.9 ml/min
Detection: (+/(-) - ESI, Fast-Switching-Mode, Scan area: 150-1500 amu, ELSD (Sedex 75,
pressure 3.5 bar, 35°C), DAD 210-400 nm
Oven Temperature: 23°C
Injection Volume: 30 µl

Mobile Phase:
A: 5mM ammonium formiate and 0.1% formic acid
B: acetonitrile/methanol = 1:1, 5 mM ammonium formiate and 0.1% formic acid (pH = 3). The details are provided in Table 2:

**Table 2**

| Details of analysis - Gradient | | |
|---|---|---|
| **Time gradient [min]** | **% A** | **% B** |
| | 85 | 15 |
| 30 | 0 | 100 |
| 30-40 | 0 | 100 |

### Example 4

### In Vitro Assay

Plant extracts from *Magnifera indica, Fumaria officinalis* and *Rumex japonicus* were prepared. The plant extracts were used in in-vitro assays using 5µg/ml *Magnifera indica* extract, 12.5 µg/ml *Fumaria officinalis* extract and 2.5 µg/ml *Rumex japonicus* extract.

### Assay conditions:

### Calcium assay based on fluorescent dye Fluo4-AM Negative Control: KH-buffer (solvent) Positive Control: ionomycin (general calcium induction) Positive Control: capsaicin (natural agonist of TRPV1)

One day prior to performing the assay, HEK293 cells stably overexpressing recombinant human TRPV1 were plated onto black-walled assay plates, at a density of 45,000 cells per well. Using the FLEX Station system the change of the cellular calcium concentration (calcium influx) induced by TRPV1 activation was monitored using the calcium sensitive fluorescent dye fluo-4 (494 nm / 516 nm (excitation / emission)). The pharmacological TRPV1 antagonist Ruthenium Red (1 µM) was used as positive control [J. Biol. Chem. 2004, 279 (34): 35741-8]. The dye-loaded stable cells in plates were placed into the fluorescence microtiter plate reader to monitor fluorescence (excitation 488 nm, emission 520 nm) change after the addition of 50µl assay buffer (118 mM NaCl; 4.7 mM KCI; 1.2 mM MgSO₄; 1.2 mM KH₂PO₄; 4.2 mM NaHCO₃; 1.3 mM CaCl₂; 10 mM HEPES (N-(2- hydroxyethyl)-piperazine-N'-2-ethanesulfonic acid) (pH: 7.4)) supplemented with an antagonist. Calcium mobilization was quantified as the change of peak fluorescence (ΔF) over the baseline level (F). The analysis was done with the software of the microtiter plate reader (FLEX Station). Potential TRPV1 antagonists (plant extracts) were tested at an effective range of 2.5 - 12.5 µg/ml (dry weight/volume). The test compounds were applied simultaneously to the agonist capsaicin. TRPV1 antagonists were tested for their antagonistic activity in the presence of 30 nM capsaicin, which is the EC_{8O} effective capsaicin concentration leading to 80 % of the maximum capsaicin-dependent TRPV1 activation.

The test result is presented in Fig. 1: Inhibition of calcium influx in HEK293 cells by TRPV1 antagonists measured by the fluorescent dye fluo-4 according to the procedure described above. Cells were stimulated with 30 nM Capsaicin (EC80) in combination with vehicle or plant extracts from *Magnifera indica* (5 µg/ml), *Fumaria officinalis* (12.5 µg/ml) or *Rumex japonicus* (2.5 µg/ml). Calcium influx induced by 30 nM capsaicin was set to 100 % (black column). The data (gray columns) are mean percentage from 2-3 experiments of residual calcium induced by 30 nM capsaicin ± SD. Statistical significance was calculated by Student's t-test, *p < 0.05, ***p < 0.001.

### Example 5

### In Vivo-Test

A study was performed with 9 volunteers who were sensitive to capsaicin. A freshly prepared solution of 31.6 ppm capsaicin (which proved to be the most appropriate concentration: clear stinging perception without erythema or burn feeling) in PBS (phosphate buffered saline) was applied to the left and right upper cheek bone area with a cotton tip with smooth rubbing. After 60 seconds all participants sensed a pronounced stinging effect. Then, an O/W-emulsion with 5 wt.% plant extracts from *Magnifera indica, Fumaria officinalis* and *Rumex japonicus* was applied on one cheek and the placebo O/W-emulsion on the other cheek (blinded samples). The stinging effect was evaluated 5 minutes later on reduction of stinging.

The three TRPV1 antagonistic plant extracts from *Magnifera indica, Fumaria officinalis* and *Rumex japonicus* inhibited the stinging sensation in 8 of 9, 7 of 9 or 8 of 9 volunteers, respectively.

The topic formulation using for the in-vivo test is represent in table 4 and the test results are presented in table 3.

**Table 3**

| In-Vivo Test | | |
|---|---|---|
| **Test No.** | **Formulation with extract of** | **% *** |
| 1 | none | 16 |
| *2* | *Magnifera indica* | 90 |
| *3* | *Fumaria officinalis* | 80 |
| *4* | *Rumex japonicas* | 80 |

| | | |
|---|---|---|
| *Percentage of individuals observing calming effect against placebo | | |

**Table 4**

| Topic formulation for In-Vivo Test (All amounts in % by weight (=% b.w.).) | | | | |
|---|---|---|---|---|
| **Compound** | **[% b.w.]** | **[% b.w.]** | **[% b.w.]** | **[% b.w.]** |
| **Plant extract** | | 10.00 | 5.00 | 1.00 |
| Aqua | 74.34 | 64.34 | 69.34 | 73.34 |
| Paraffinum Liquidum (Hansen & Rosenthal KG) | 8.00 | 8.00 | 8.00 | 8.00 |
| Isopropyl palmitate (Azelis Kosmetik GmbH) | 4.00 | 4.00 | 4.00 | 4.00 |
| Cetearyl Isononanoate (Azelis Kosmetik GmbH) | 4.00 | 4.00 | 4.00 | 4.00 |
| Glycerin (Fauth & Co.KG) | 3.00 | 3.00 | 3.00 | 3.00 |
| Methyl Glucose Sesquiisostearate (Univar GmbH) | 3.50 | 3.50 | 3.50 | 3.50 |
| Sorbitan stearate (Univar GmbH) | 3.50 | 3.50 | 3.50 | 3.50 |
| Olea European Fruit Oil (Gustav Heess) | 1.00 | 1.00 | 1.00 | 1.00 |
| Cetearyl alcohol (Azelis Kosmetik GmbH) | 0.30 | 0.30 | 0.30 | 0.30 |
| Sodium Carbomer (NEOCHEM) | 0.30 | 0.30 | 0.30 | 0.30 |
| Allantoin (Azelis Kosmetik GmbH) | 0.20 | 0.20 | 0.20 | 0.20 |
| Panthenol, Propylene Glycol (Brenntag GmbH) | 0.30 | 0.30 | 0.30 | 0.30 |
| Methylparaben, Butylparaben, Ethylparaben, Isobutylparaben, Propylparaben, Phenoxyethanol (Cosnaderm Chemische Rohstoffe GmbH) | 1.00 | 1.00 | 1.00 | 1.00 |
| Tocopherol, Hydrogentaed Palm Glycerides citrate (Azelis Kosmetik GmbH) | 0.05 | 0.05 | 0.05 | 0.05 |
| Sodium hyalruonate | 0.01 | 0.01 | 0.01 | 0.01 |

In the following tables various formulation examples are provided.

**Table 5**

| Skin-soothing lotion with plant extract O/W | |
|---|---|
| **Compound** | **[% b.w.]** |
| **Plant extract** | 0.8 |
| Abil 350 (Degussa-Goldschmidt) (INCI: Dimethicone) | 2.0 |
| Allantoin (Merck) | 0.2 |
| Aqua- Ceramide (Kao) (INCI: Cetyloxypropyl Glyceryl Methoxypropyl Myristamide) | 0.1 |
| Carbopol Ultrez-10 (Noveon) (INCI:Carbomer) | 0.1 |
| Dracorin GMS (Symrise) (INCI:Glycerylstearate) | 2.0 |
| Dragocid liquid (Symrise) (INCI: Phenoxyethanol, Methyparabene, Ethylparabene, Butylparabene, Propylparabene, Isobutylparabene) | 0.8 |
| Dragoxat EH (INCI: Ethylhexyl- ethylhexanoat) | 3.0 |
| Emulsiphos (Symrise) (INCI: Potassium Cetylphosphate, Hydrogenated Palm-glycerides) | 2.0 |
| Extrapone Green Tea GW (Symrise) (INCI: Glycerine, Water (Aqua), Camellia Sinensis Leaf Extract) | 0.2 |
| Extrapone Rosemary GW (Symrise) (INCI: Glycerine, Water (Aqua), Rosmarinus officinalis Leaf Extract) | 0.3 |
| Glycerol 85% (INCI: Glycerine) | 2.0 |
| Isodragol (Symrise) (INCI: Triisononanoin) | 2.0 |
| Keltrol RD (CP-Kelco) (INCI: Xanthan Gum) | 0.1 |
| Lanette O (Cognis) (INCI: Cetearyl Alcohol) | 3.0 |
| PCL Liquid 100 (Symrise) (INCI: Cetearyl Ethylhexoate) | 5.0 |
| PCL Solid (Symrise) (INCI: Stearyl Heptanoate, Stearyl Caprylate) | 2.0 |
| 1,2-Propylene Glycol 99P GC (INCI: Propylene-glycole) | 5.0 |
| Pseudoceramide 391 (INCI: N-(1-Hexadecanoyl)-4-hydroxy-L-proline (1-hexadecyl)ester | 0.1 |
| Sodium hydroxide (10% sol.) | 0.3 |
| SymMatrix (Symrise) (INCI: Maltodextrin, Rubus Fructicosus (Blackberry) Leaf Extract) | 0.1 |
| Fragrance | 0.3 |
| Water (Aqua) | to 100 |

**Table 6**

| Aftersun Lotion | |
|---|---|
| **Compound** | **[% b.w.]** |
| **Plant extract** | 1.1 |
| Abil 350 (Degussa-Goldschmidt) (INCI: Dimethicone) | 1.0 |
| Ginger CO2 Extract (Flavex) (NCI: Zingiber Officinale (Ginger) Root Extract | 0.003 |
| Allantoin (Merck) | 0.1 |
| Aloe Vera Gel Concentrate 10/1 (Symrise) | 3.0 |
| Sodium Ascorbyl Phosphate (EMD Chemicals) (INCI: Sodiumascorbyl-phosphate) | 1.0 |
| Butylene Glycol | 5.0 |
| Cetiol OE (Cognis) (INCI: Dicaprylylether) | 4.0 |
| Cetiol SB 45 (Cognis) (INCI: Butyrospermum Parkii (Shea Butter) | 1.0 |
| D-Panthenol (BASF) (INCI: Panthenol) | 1.0 |
| Dragocid liquid (Symrise) (INCI: Phenoxyethanol, Methyparabene, Ethylparabene, Butylparabene, Propylparabene, Isobutylparabene) | 0.7 |
| Frescolat MGA (Symrise) (INCI: Menthone glycerolacetal) | 0.8 |
| Glycerol 85% (INCI: Glycerine) | 4.0 |
| Lara Care A-200 (Rahn) (INCI: Galactoarabinan) | 0.3 |
| Pemulen TR-2 (Noveon) (INCI: Acrylates/C10-30 Alkylacrylate Crosspolymer) | 0.3 |
| Sodium hydroxide (10% sol.) | 0.6 |
| Tegosoft PC 31 (Degussa) (INCI: C12-15-Alkylbenzoate) | 5.0 |
| Tocopherol Acetate (DSM Nutritional Products) | 0.5 |
| Fragrance | 0.3 |
| Water (Aqua) | to 100 |

**Table 6**

| W/O night creme | |
|---|---|
| **Compound** | **[% b.w.]** |
| **Plant extract** | 1.75 |
| (Alpha)-Bisabolol, neutral (Symrise) | 0.2 |
| Aloe Vera Gel Concentrate 10/1 (Symrise) | 3.0 |
| Alugel 34 TH (Baerlocher) (INCI: Aluminiumstearate) | 1.0 |
| Ceramide SL (Sino Lion) (INCI: hydroxyethyl Palmityl Oxyhydroxypropyl Palmitamide) | 0.1 |
| Dragocid liquid (Symrise) (INCI: Phenoxyethanol, Methyparabene, Ethylparabene, Butylparabene, Propylparabene, Isobutylparabene) | 0.8 |
| Dragosan W/O Liquid (symrise) (INCI: Polyglyceryl-3-Polyricinoleate, Sorbitan-isostearate) | 1.0 |
| Dragosan W/O Liquid (symrise) (INCI: Sorbitan, Isostearate, Hydrogenated Castor Oil, Ceresin, Beeswax (Cera Alba)) | 6.0 |
| Extrapone Witch Hazel Distillate colourless (Symrise) (INCI: Propylene-glycole, Hamamelis Virginiana (Witch Hazel) Water (Aqua), Hamamelis Virginiana (Witch Hazel) Extract) | 1.0 |
| Glycerol 85% (INCI: Glycerine) | 2.0 |
| Karion F (Merck) (INCI: Sorbitol) | 2.0 |
| Magnesium Chloride (Merck) | 0.7 |
| Oxynex 2004 (Merck) (INCI:Butylhydroxy-toluol) | 0.1 |
| PCL Liquid (Symrise) (INCI: Cetearyl Ethylhexanoate, Isopropyl-myristate) | 12.0 |
| Retinyl Palmitate in Oil (DSM Nutritional Products) | 0.2 |
| Sun Flower (INCI: Helianthus Annuus (Sunflower) Sweed Oil)) | 5.0 |
| Sweet Almond Oil (Wagner (INCI: Prunus dulcis) | 5.0 |
| SymMatrix (Symrise) (INCI: Maltodextrin, Rubus Fructicosus (Blackberry) Leaf Extract) | 1.0 |
| Tocopherol Acetate (DSM Nutritional Products) | 3.0 |
| Fragrance | 0.4 |
| Water (Aqua) | to 100 |

**Table 7**

| Micro-emulsion gel | |
|---|---|
| **Compound** | **[% b.w.]** |
| **Plant extract** | 1.95 |
| Glycerin isostearate | 1.80 |
| Octoxyglycerin | 1.00 |
| Ceteareth-15 | 5.20 |
| PEG-150 Distearate | 1.00 |
| Aluminium chlorohydrate | 5.00 |
| Isotridecylisononanoate | 3.30 |
| Cyclomethicone | 6.60 |
| Fragrance | 0.55 |
| Water | Add to 100 |

## Claims

1. A composition obtainable by the method of extracting plant materials selected from the group consisting of *Mangifera indica* and/or *Fumaria officinalis* and/or *Rumex japonicus.*

2. A cosmetic formulation comprising
(i) the composition obtainable according to claim 1 and
(ii) one or more cosmetically acceptable carriers and/or solvent,
wherein the composition in (i) is provided in a dosage form providing an amount effective to prevent, treat or alleviate symptoms of disease, disorder or conditions associated with TRPV1 activity.

3. A pharmaceutical preparation comprising
(i) the composition obtainable according to claim 1 and
(ii) one or more pharmaceutically acceptable carriers and/or solvent,
wherein the composition in (i) is provided in a dosage form providing an amount effective to prevent, treat or alleviate symptoms of disease, disorder or conditions associated with TRPV1 activity.

4. The pharmaceutical preparation of Claim 3, wherein the preparation comprises further additives selected from the group comprising of: anti-inflammatory agents, anti-scarring agents, anti-psoriatic agents, anti-proliferative agents, anti-fungal agents, anti-viral agents or anti-septic agents or mixtures thereof.

5. The cosmetic formulation of Claim 2 or the pharmaceutical preparation of Claim 3, wherein said compositions or preparations are administered by topical, transdermal or oral application.

6. A method for treating or preventing a condition involving activation of TRPV1 or for which reduced TRPV1 activity can reduce severity of symptoms, comprising administering an effective amount of at least one plant extract consisting of *Mangifera indica and*/*or Fumaria officinalis and*/*or Rumex japonicus.*

7. The method of Claim 6, wherein the treatment, prevention or alleviation of symptoms is associated with TRPV1 activity which is selected from the group consisting of acute pain, chronic pain, touch sensitivity, itching sensitivity, inflammation, fever, hot flashes, rheumatoid arthritis.

8. The method of Claim 6, wherein the effective amount is an amount that is sufficient to modulate and reduce the TRPV1 receptor activity by at least 30 percent.

9. Use of a composition comprising the extract of plant materials selected from the group consisting of *Mangifera indica* and/or *Fumaria officinalis* and/or *Rumex japonicus* in the manufacture of a pharmaceutical preparation or cosmetic formulation for preventing or treating diseases or conditions that are alleviated by blockade of one or more types of TRP channel.

10. The use according to claim 9 wherein the TRP channel is TRPV1.

11. The use according to claim 9 and/or 10, wherein the composition is a plant extract which is obtainable according to claim 1.

12. The use according to any of the claims 9 to 11, wherein the diseases or conditions that are alleviated by blockade of one or more types of TRP channel is selected from the group consisting of stinging, burning, tingling, tickling and skin tightness, acute pain, chronic pain, touch sensitivity, itching sensitivity, inflammation, fever, hot flashes, rheumatoid arthritis.

13. Use of a composition comprising the extract of plant materials selected from the group consisting of *Mangifera indica* and/or *Fumaria officinalis* and/or *Rumex japonicus* as an antagonistic agent for TRPV1.

14. The use according to claim 13 wherein the antagonistic agent for TRPV1 reduces skin irritation selected from the group consisting of stinging, burning, tingling, tickling and skin tightness.

15. The use according to claim 13 and/or 14, wherein the antagonistic agent for TRPV1 is used in an amount that is sufficient to modulate and reduce the TRPV1 receptor activity by at least 30 percent.
